# EUROPEAN PATENT SPECIFICATION

(11) **EP 4 344 676 B1**
(45) Date of publication and mention of the grant of the patent: **09.12.2026**
(21) Application number: 23202703.7
(22) Date of filing: 14.12.2015
(51) Int. Cl.: A61F 2/24

(54) **PROSTHETIC VALVE AND DEPLOYMENT SYSTEM**
PROTHETISCHE KLAPPE UND EINSETZSYSTEM
VALVULE PROTHÉTIQUE ET SYSTÈME DE DÉPLOIEMENT

(30) Priority: 14.12.2014 US 201462091595 P; 30.03.2015 US 201562139907 P
(43) Date of publication of application: 03.04.2024
(62) Document number(s) of the earlier application(s) in accordance with Art. 76 EPC: 15869467.9 / 3 229 738
(73) Proprietor: Trisol Medical Ltd., 2069200 Yokneam (IL)
(72) Inventor: VATURI, Mordehay, 5591228 Ganei-Tikva (IL); GOLLAN, Tal, Ramat-Gan 5258434 (IL)
(74) Representative: Dennemeyer & Associates S.A.

(56) References cited:
- FR-A1- 2 883 721
- US-A1- 2005 228 495
- US-A1- 2010 217 381

## Description

### RELATED APPLICATION/S

This application claims the benefit of priority under 35 USC §119(e) of U.S. Provisional Patent Application No. 62/091,595 filed 14 December 2014 and U.S. Provisional Patent Application No. 62/139,907 filed 30 March 2015.

### FIELD AND BACKGROUND

Embodiments disclosed herein generally relate to a prosthetic valve and, more particularly, but not exclusively, to a prosthetic replacement for a tricuspid valve and catheter delivery system.

US Patent Publication No. 20050228495 to Macoviak discloses a valve prosthesis that, "is sized and configured to rest within a blood path subject to antegrade and retrograde blood flow. A trestle element on the prosthesis extends across the blood path. A leaflet assembly is suspended from the trestle element and extends into the blood path in alignment with blood flow. At least one mobile leaflet member on the leaflet assembly is sized and configured to assume orientations that change according to blood flow direction. The mobile leaflet member has a first orientation that permits antegrade blood flow and a second orientation that resists retrograde blood flow. The valve prosthesis, when implanted in a heart chamber or great vessel, serves to supplement and/or repair and/or replace native one-way heart valve function."

US Patent No. 4561129 to Arpesella "relates to a 'biological' valve suitable for surgical treatment of cardiopathies, composed of a supporting ring having a diametrically-disposed element which functions as a support for the hinge of the two flaps which alternately allow and prevent flow through the device. It is designed in such a way that the angle formed by these flaps in the resting position conditions both the height of the prosthesis as well as the mechanical behavior of the aforementioned flaps."

US Patent Publication No. 20110264206 to Tabor relates to "prosthetic valves having sealing members on the external surface thereof. The prosthetic heart valves of the present invention are preferably delivered by catheter directly through the apex of the heart or by other close range transcatheter delivery methods..."

US Patent Publication No. 20110040374 to Goetz et al. relates to "A replacement valve for implantation centrally within the orifice of a malfunctioning native heart valve. The valve is designed for minimally invasive entry through an intercostal opening in the chest of a patient and an opening in the apex of the human heart. The replacement valve includes either a separate anchor or a combined anchor that folds around the malfunctioning native valve leaflets, sandwiching them in a manner so as to securely anchor the replacement valve in a precise, desired location."

International Patent Publication No. WO 2013076724 to Vaturi relates to "A method and a device for implantation in or near an annulus of a tricuspid valve comprising at least one blood flow control element adapted to capture a volume of blood therein."

### SUMMARY

The invention is as set forth in the appended claims.

This summary is provided to introduce a selection of concepts in a simplified form that are further described below in the Detailed Description. This summary is not intended to identify key features or essential features of the claimed subject matter, nor is it intended to be used to limit the scope of the claimed subject matter.

Unless otherwise defined, all technical and/or scientific terms used herein have the same meaning as commonly understood by one of ordinary skill in the art to which the invention pertains. Although methods and materials similar to those described herein can be used in the practice or testing of embodiments of the invention, exemplary methods and/or materials are described below. In case of conflict, the patent specification, including definitions, will control. In addition, the materials, methods, and examples are illustrative only and are not intended to be necessarily limiting.

### BRIEF DESCRIPTION OF THE SEVERAL VIEWS OF THE DRAWINGS

Some embodiments are herein described, by way of example only, with reference to the accompanying drawings. With specific reference now to the drawings in detail, it is stressed that the particulars shown are by way of example and for purposes of illustrative discussion of embodiments of the invention. In this regard, the description taken with the drawings makes apparent to those skilled in the art how embodiments of the invention may be practiced.

In the drawings:
Figure 1 is a state diagram of a prosthetic valve and/or a delivery system in accordance with some embodiments;
Figures 2A and 2B are block diagrams of prosthetic valves in accordance with some embodiments;
Figure 3 is a block diagram of a prosthetic valve and/or a delivery system in accordance with some embodiments;
Figure 4 is a flow chart illustrating functioning of a prosthetic valve in accordance with some embodiments;
Figure 5 is a chart illustrating deployment of a prosthetic valve in accordance with some embodiments;
Figure 6 is a perspective illustration of a prosthetic valve with the frame expanded and the valve open in accordance with the current invention;
Figure 7 is a perspective illustration of a prosthetic valve with the frame expanded and the valve closed in accordance with some embodiments;
Figure 8A is a side view illustration of a prosthetic valve with the frame expanded and the valve open in accordance with some embodiments;
Figure 8B is a side view illustration of a prosthetic valve with the frame expanded and the valve closed in accordance with some embodiments;
Figure 9A is a top view illustration of a prosthetic valve with the frame expanded and the valve open in accordance with some embodiments;
Figure 9B is a top view illustration of a prosthetic valve with the frame expanded and the valve closed in accordance with some embodiments;
Figure 9C is a top view illustration of a prosthetic valve with the frame expanded and the valve open in accordance with some embodiments;
Figure 9D is a top view illustration of a prosthetic valve with the frame expanded and the valve closed in accordance with some embodiments;
Figure 9E is a perspective view illustration of a prosthetic valve with the frame expanded and the valve closed in accordance with some embodiments;
Figure 10 is an illustration of a tricuspid annulus showing positioning of a prosthetic valve in accordance with some embodiments;
Figure 11 is a perspective illustration of a prosthetic valve with the frame collapsed in accordance with some embodiments;
Figure 12 is a perspective illustration of a prosthetic valve with the frame collapsed and ventricle anchors deployed in accordance with some embodiments;
Figure 13 is a close up perspective illustration of a prosthetic valve and deployment system with the frame expanded and the valve open in accordance with some embodiments;
Figure 14 is a close up perspective illustration of a prosthetic valve and deployment system with the frame collapsed in accordance with some embodiments;
Figure 15 is a perspective illustration of a prosthetic valve and deployment system with the frame collapsed in accordance with some embodiments;
Figure 16 is a close up perspective illustration of a prosthetic valve and deployment system with the frame collapsed and ventricle stabilizers deployed in accordance with some embodiments; and
Figure 17 is a close up perspective illustration of deployment of a prosthetic valve in accordance with some embodiments.

### DESCRIPTION OF SPECIFIC EMBODIMENTS

### OVERVIEW

Aspects of disclosed embodiments relates to a prosthetic valve and, more particularly, but not exclusively, to a prosthetic replacement for a tricuspid valve and catheter delivery system for the prosthetic valve replacement.

An aspect of some embodiments relates to a peripheral flow prosthetic valve. Optionally the valve includes a diaphragm supported by a connection to the frame along a length of the frame. When the valve is open, the diaphragm is optionally collapsed. For example the two sides of the diaphragm may collapse toward the center. Optionally, the portion of the diaphragm connected to the frame is held expanded and/or open along the length of said connection to the frame. For example, flow from an upstream region to a downstream region causes said diaphragm to collapse and/or opens said valve. For example, flow from a downstream region to an upstream region is trapped in the expanded and/or open portion of the diaphragm and/or expands the diaphragm and/or closes the valve.

Optionally the apex of the diaphragm faces in the upstream direction. Optionally the apex of the diaphragm may remain upright when the dome collapses. When the valve is open, flow optionally passes between the diaphragm and the periphery of the orifice. For example, flow may pass the diaphragm on at least two opposing sides thereof. When the valve is closed the free edge of the diaphragm may optionally expand against the periphery of the orifice and/or the frame and/or a skirt connected to the frame. Expanding the diaphragm against the periphery of the orifice optionally closes the valve and/or or impedes flow through the orifice to flow. For example fluid pressure may push the flexible diaphragm against the inner walls of orifice and/or the frame.

In some embodiments, the diaphragm may be dome shaped when the diaphragm is spread and/or when the valve is closed to flow. For example the concave side of the diaphragm may be facing downstream. Optionally the apex of the dome extends upstream beyond the frame.

In some embodiments, the frame may be flexible. For example, a tricuspid orifice and/or the frame may take an elliptical and/or half moon shape during systole and/or a circular shape during diastole. The closing of the valve may be substantially unaffected by the flexing of the frame. Optionally, the valve allows controlled regurgitation. For example the dome shaped membrane optionally captures a predetermined quantity of blood during systole. Some or/all of the captured blood is optionally returned during diastole.

In some embodiments, the diaphragm is made of a single piece of material. Alternatively or additionally the diaphragm may be made of multiple pieces. Optionally the diaphragm may include one or more leaflets. Optionally, the diaphragm material may be a flexible biocompatible polymer, pericardium and/or include bovine and/or porcine pericardium.

In some embodiments, the diaphragm may fill with fluid like parachute, a sail and/or a balloon. For example, during systole, the diaphragm may take a dome and/or parabolic shape. For example the shape of the dome may change with deformations of the frame, for example between systole and diastole.

The supports of the diaphragm on the periphery hold the diaphragm in a ready position during flow through the valve. For example, the supports may hold the diaphragm partially extended when the valve is open. For example, for a tricuspid prosthesis, attachment to the periphery of a tricuspid orifice may hold a portion of parachute type diaphragm expanded during diastole. The partially expanded diaphragm optionally catches fluid during systole, further expanding the diaphragm and/or closing the valve and/or closing off the orifice and/or impeding flow through the orifice.

In some embodiments, an apex of the diaphragm may extend past the edge of a frame of the valve. For example the apex of the diaphragm may extend in an upstream direction from the frame ranging between 0 to 12 mm and/or between 12 to 22 mm and/or between 22 to 30 mm. In some embodiments, the unstressed length of the edge of the diaphragm may be larger than the perimeter of the orifice. Additionally or alternatively, the edge of the diaphragm may be smaller than the perimeter of the orifice. The edge of the diaphragm optionally extends against the perimeter of the orifice under fluid pressure and/or closes the valve. For example the length of the edge of the diaphragm may range between 0.5 to 1 times the perimeter of the orifice and/or between 1 to 1.2 times the perimeter of the orifice and/or between 1 to 1.2 times the perimeter of the orifice and/or between 1.2 to 1.6 times the perimeter of the orifice and/or between 1.6 to 3 times the perimeter of the orifice. In some embodiments, the surface area of one side of the diaphragm may be greater than the area of a circle having the same perimeter length as the diaphragm. For example the surface area of the diaphragm may range between 1.5 to 3 times the area of a circle having the same perimeter length as the diaphragm and/or between 3 to 5 times the area of a circle having the same perimeter length as the diaphragm and/or between 5 to 10 times the area of a circle having the same perimeter length as the diaphragm. In some embodiments, the surface area of the diaphragm may be greater than the cross sectional area of the orifice. For example the surface area of the diaphragm may range between 1.5 to 3 times the cross sectional area of the orifice and/or between 3 to 5 times the cross sectional area of the orifice and/or between 5 to 10 times the cross sectional area of the orifice and/or more than 10 times the cross sectional area of the orifice.

In some embodiments, the frame may include one or more support elements extending into the orifice. For example the support elements may extend between 0 to 2 mm and/or between 2 to 5 mm and/or between 5 to 15 mm upstream of the peripheral portion of the frame. Alternatively or additionally, a support element may be surrounded by and/or within the peripheral portion of the frame. In some embodiments, the support elements do not reach the center of the orifice and/or are connected to only one side of the orifice. For example a support element may have cantilever geometry, for example only one side of the support element may be attached to the periphery of the frame and/or periphery of the orifice. Optionally the support elements may extend 0 to 3 mm and/or 3 to 6 mm and/or 6 to 10 mm from the periphery towards the center of the orifice. Alternatively or additionally a support element may cross the orifice and/or may be attached at both ends and/or at multiple points to the periphery of the frame and/or orifice. Optionally a support element may be located on the upstream side and/or the downstream side of the diaphragm. Optionally the diaphragm is attached (for example by sutures) to one or more support elements.

An aspect of some embodiments relates to a prosthetic valve with controlled regurgitation. Optionally the valve includes a parachute diaphragm and/or a balloon diaphragm and/or a sail diaphragm that fills with blood when flow is blocked. For example, the valve may be used as a tricuspid prosthesis. The diaphragm may fill with between 0 to 10 ml and/or between 10 to 20 ml and/or between 20 to 30 ml and/or 30 to 40 ml of fluid as it seals off an orifice impeding and/or inhibiting and/or preventing further regurgitation. For example, for a tricuspid prosthesis during systole, the parachute may fill with a volume between 0 to 10% and/or 10 to 20% and/or 20 to 30% and/or 30 to 40% of the volume of the ventricle to which the valve is attached. Part and/or all of the blood that filled the parachute is optionally returned to the ventricle during diastole.

One issue that may occur with defective native valves is regurgitation. While there is excess regurgitation, the cardiac muscle may sometimes contract with a greater increased force and/or volume. In some cases increased stroke may compensate for the regurgitation and/or sustain cardiac output. This process gradually has been hypothesized to lead in some cases to cardiac muscle remodeling and/or eventually to systolic dysfunction due to reduced contractility. In some cases remodeling may be followed by even greater regurgitation, for example due to dilatation of the ventricle and/or increased tethering of the valve and/or pulmonary edema and/or systemic venous congestion.

In some cases, an abrupt cease of regurgitation may lead and/or an acute increase in afterload on the ventricle. In some cases the consequence may be deleterious to the ventricle. Optionally some embodiments of a prosthetic valve may reduce regurgitation and/or allow mild regurgitation (for example hemodynamically insignificant to the heart). Optionally the controlled and/or mild regurgitation may ease the afterload on the ventricle. Optionally, during systole the volume of the diaphragm may effectively be added to the volume of the ventricle. For example, temporarily increasing the volume of the ventricle may reduce the load on the ease the afterload on the ventricle.

In some embodiments controlled regurgitation may be supplied by one or more holes in the diaphragm. A hole may be located at the center of the diaphragm and/or non-centered. The total area of the holes may range, for example between 0.2 mm² and 0.8 mm² and/or between 0.8 mm²and 2 mm²and/or between 2 mm² and 15 mm² and or between 15 mm² and 25 mm².

An aspect of some embodiments relates to a prosthetic valve with a flexible frame. Optionally the frame may change shape with the annulus of the natural orifice. For example in a tricuspid annulus, the frame may take an oval shape during systole and/or a more circular shape during diastole. Optionally the valve includes a parachute like diaphragm that may be pushed against the wall of the orifices (for example the inner walls of the frame and/or a skirt). Pushing the diaphragm against the wall of the orifice optionally closes the orifice to back flow and/or regurgitation. For example, fluid pressure may push the diaphragm evenly against the inner walls of the frame and/or orifice. For example, fluid pressure may be applied evenly and/or close the valve evenly substantially independent of the geometry of the orifice. Optionally, the frame may not include a stiff member limiting the length of an axis of the frame's cross section (for example a transverse axis). For example, the frame may not include any stiff crosspiece. Optionally the frame may not be limited by any stiff member connected on opposing sides of a central region of the frame. For example, the frame may define a simply connected interior region and/or a single flow channel. The diaphragm is optionally suspended across the frame dividing said simply connected flow channel into at least two channels. For example, without the diaphragm, a cross section of the frame and/or a two dimensional projection of the cross section of the frame may include a single simply connected interior region. For example, with the diaphragm, a cross section of the frame and diaphragm and/or a two dimensional projection of the cross section of the frame and diaphragm may include at least two disconnected interior regions.

In some embodiments, the diaphragm may be connected to and/or contact the frame at nearly tangential angle (for example ranging between 0 to 5 degrees of tangential and/or between 5 and 15 degrees and/or between 15 and 30 degrees). This may lead to reduced radial forces on the frame and/or allow use of a more compliant and/or flexible frame. This may sometimes may fluid pressure more effective in closing the valve. For example the radial forces on the frame and/or on the closing interface of the valve may be substantially pressure forces. Optionally, the net force and/or the pressure force on the diaphragm along a portion of inner wall of the frame (for example along between 50 and 80% of the non-attached interface between the diaphragm and the frame and/or over more than 80% of the non-attached interface) may be substantially perpendicular to the frame (for example ranging between 0 to 5 degrees of perpendicular and/or between 5 and 15 degrees and/or between 15 and 30 degrees).

Optionally the length (for example the longitudinal axis and/or the maximum and/or mean wall height) of the frame may be short (for example ranging between 5 to 15 and/or between 15 to 20 mm and/or between 20 to 25 mm and/or between 25 to 30 mm and/or between 30 to 45 mm and/or between 45 to 70 mm). For example the axial length of the frame may range between 20 to 30% of its outer perimeter and/or between 30 to 40% and/or between 40 to 50% of its outer perimeter (for example the outer perimeter may be defined as the perimeter of the frame, for example its circumference, at the ventricle end and/or the minimum perimeter of the orifice in which the frame is to be inserted. For a tricuspid prosthesis, the frame is optionally short enough to avoid blocking the right ventricular outflow tract (RVOT). Optionally the frame is long enough for the diaphragm to reliably close against the inner walls of the orifice.

In some embodiments, the diaphragm may include a smooth curved dome shape, for example an ellipsoid dome optionally including a cylindrical section. The volume of the diaphragm would optionally be for example π*ab*(*h* + 2c/3) where *a* and *b* are the long and short radii of the cross section and h is the height of the cylindrical portion and c is the height of the ellipsoid section. For example a circular dome of radius *a=b*=17 mm and height c=25 mm would have a volume of approximately 15 ml. If a diaphragm had for example an additional right circular cylindrical section of height 5 mm it would have a volume of approximately 20 ml. For a large regurgitation volume and/or large surface area of the diaphragm the length of the frame may be increase (for example in order to improve closing of the valve and/or avoid the inversion of the diaphragm).

In some embodiments, the frame may have an irregularity. For example for a tricuspid prosthesis, the frame may be flattened along a length of the perimeter ranging between 0.5 to 2 mm and/or between 2 to 6 mm and between 6 to 10 mm.•For example the flattening may be in a region where the frame comes near the septal wall of the heart. For example, the flattening may be in the vicinity of a septal leaf ventricle extension.

An aspect of some embodiments relates to a delivery system for a prosthetic valve. For example, the delivery system may include a guide wire and/or a hollow tube through which the guide wire passes. The delivery system may optionally include a knurl and/or tip. In some embodiments, tip may be blunt. For example blunt enough to prevent damage to the vessel wall. The hollow tube is optionally threaded into the tip. Optionally the guide wire locks the tube and the tip together. For example, the guide wire may prevent the hollow tube from disconnecting from the guide wire and/or prevents damage to vessel walls as the device is navigated to the treatment area. For example the guide wire may pass through an opening in the knurl. While guide wire passes through the knurl the hollow tube and/or the prosthesis are optionally be prevented from being released. Optionally, the knurl may have a blunt shape, for example to prevent damage to the vessel wall. The guide wire and/or tube is optionally is threaded through the knurl. Threading the wire and/or tube through the knurl optionally locks the hollow tube and/or knurl and/or wire together.

In some embodiments, the guide wire and/or hollow tube may pass through a channel of the prosthesis. In some embodiments an extender (for example including a shaft and/or a wire and/or a tube and/or a chord) may pass through a second channel of the prosthesis. For example the extender may be connected to and/or retain the knurl.

In some embodiments, objects passing through multiple channels of the prosthesis may distribute the forces on the prosthesis. For example, passing objects through multiple channels may distribute forces more evenly. Alternatively or additionally passing objects through multiple channels may balance and/or reduce stresses on a vulnerable part of the prosthesis. For example, passing the guide wire through a first channel of a peripheral flow valve and an extender through a second channel may protect a delicate central diaphragm from unbalanced forces.

In some embodiments, the prosthesis may be positioned in the heart by transcatheter and/or percutaneous delivery.

Before explaining at least one embodiment of the invention in detail, it is to be understood that the invention is not necessarily limited in its application to the details of construction and the arrangement of the components and/or methods set forth in the following description and/or illustrated in the drawings and/or the Examples. The invention is capable of other embodiments or of being practiced or carried out in various ways.

### States of a prosthesis

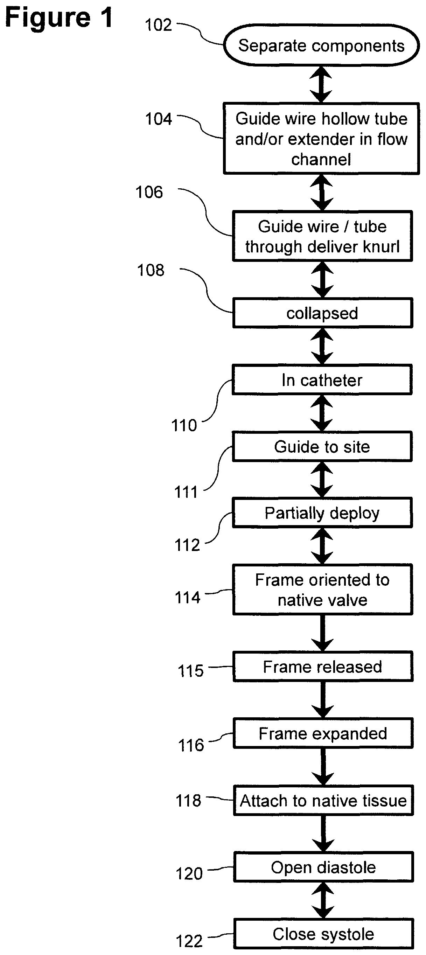

Referring now to the drawings, *FIG. 1* is a state diagram of a prosthetic valve and/or delivery system in accordance with some embodiments. For example, the valve and delivery system may be assembled and/or inserted into a patient (for example using a catheter and/or percutaneous delivery. In some embodiments, deployment of the prosthesis may include placing the prosthesis and/or orienting the prosthesis and/or attached the prosthesis to the native tissue. Optionally after delivery the delivery system is released and/or retrieved.

In some separate components **102** of the prosthesis and/or delivery system may be assembled. For example a guide wire and/or a hollow tube and/or an extender may be reversible inserted **104** through one or more flow channels of the prosthesis. A delivery knurl is optionally attached to the distal portion of the extender. For example a hollow tube may be inserted into one channel of the prosthesis and/or a guide wire may be inserted through the hollow tube. For example an extender may be inserted through the one channel and/or through a second channel. In some embodiments the distal end of the guide wire and/or the hollow tube may be reversibly threaded **106** through an opening in the delivery knurl.

In some embodiments, the prosthesis may have a collapsed **108** state. For example the prosthesis may be reversibly collapsed over the hollow tube, guide wire and/or extender. Optionally in the collapsed **108** state, the prosthesis may be reversibly fit into a catheter **110,** for example, for delivery to an affected organ.

In some embodiments, the valve and/or the catheter may be guided **111** to the implantation site.

In some embodiments, the prosthesis may be partially deployed **112** from the catheter. For example in the partially deployed **112** state, a ventricle anchor may be deployed from the catheter. In some embodiments, deployment of an anchor inside a patient may be irreversible and/or difficult to reverse. Alternatively or additionally, deploying of an anchor may be reversible. The ventricle anchors are optionally used to reversibly guide to prosthesis to a position oriented **114** to the native structure (for example for a valve prosthesis to the native valve).

In some embodiments, the prosthesis may be released **115** from the catheter opened into an expanded **116** state. The expanded **116** prosthesis may be attached **118** to the native tissue. Attachment may include, for example placing anchors and/or coapting a ring to native tissue. Optionally expansion and/or attachment inside a patent may be irreversible and/or difficult to reverse. Alternatively or additionally expansion and/or attachment may be reversible.

In some embodiments, a valve prosthesis may have an open **120** and/or a closed **122** state. For example, a tricuspid valve prosthesis may be open **120** during diastole and/or closed **122** during systole.

### A prosthesis

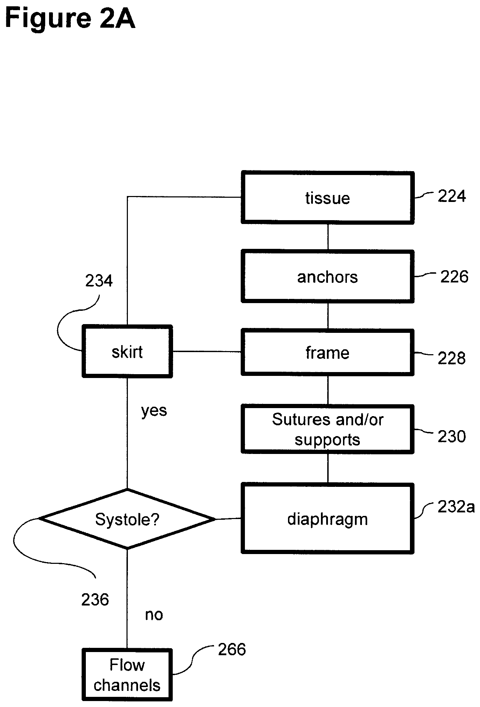

Referring now to the drawings, *FIG. 2A* is a block diagram of a prosthetic valve in accordance with some embodiments. In some embodiments, the prosthesis optionally includes a frame **228** that is attached to native tissue **224.** Optionally frame **228** is attached to the tissue **224** by means of one or more anchors **226** and/or by means of a coaptive skirt **234.** The frame is optionally attached to a diaphragm **232a.** For example the frame may be attached to the diaphragm **232a** via sutures (for example on the periphery of the frame) and/or one or more supports and/or a coaptive skirt **234.** For example, for a tricuspid valve prosthesis, the diaphragm may close against the frame during systole, impeding flow through the valve and/or the diaphragm may be distanced from the frame during diastole opening one or more peripheral flow channels **266.**

Optionally diaphragm **232a** includes a pericardium. Optionally diaphragm **232a** includes one or more leaflets. Optionally the thickness of diaphragm **232a** ranges between 0.1 to 0.5mm.

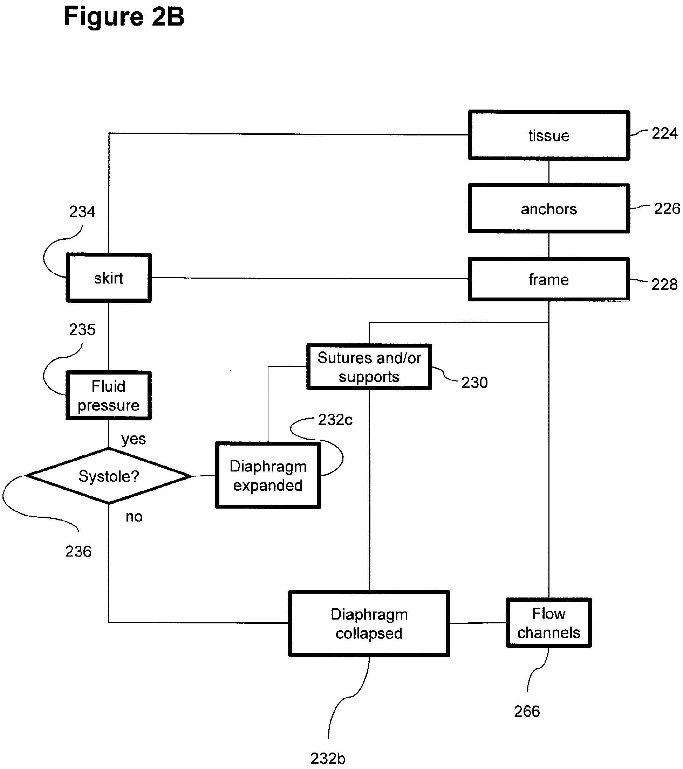

Referring now to the drawings, *FIG. 2B* is a block diagram of a prosthetic valve in accordance with some embodiments. In some embodiments, the prosthesis optionally includes a frame **228** that is attached to native tissue **224.** Optionally frame **228** is attached to the tissue **224** by means of one or more anchors **226.** The frame is optionally attached to a diaphragm (for example, an attached portion of the diaphragm may be attached to a portion of the frame in an expanded **232c** and/or a collapsed **232b** state). Optionally, the frame may be attached to the diaphragm on the periphery of the orifice, for example via sutures **230** and/or one or more supports and/or a skirt **234.**

In some embodiments, the diaphragm in the expanded state **232c** may close the valve. Optionally, an unattached portion of the diaphragm in the expanded state **232c** may press against frame **228** and/or a skirt **234.** For example a tricuspid prosthesis diaphragm may be in an expanded state **232c** during systole **236.** Closing the valve optionally impedes flow through the valve. Optionally, fluid pressure **235** may supply a force pushing the diaphragm in an expanded state **232c** against the frame. Optionally, during diastole (for example not during systole **236)** the diaphragm in a collapsed state **232b** may be distanced from the frame. For example, fluid pressure **235** may distance the diaphragm in the collapsed state **232b** from frame **228.** Distancing the diaphragm in the collapsed state **232b** from the frame optionally opens one or more peripheral flow channels **266.** For example, flow channels **266** may be located between frame **228** and an unattached portion of the diaphragm in the collapsed state **232b**.

### A delivery system

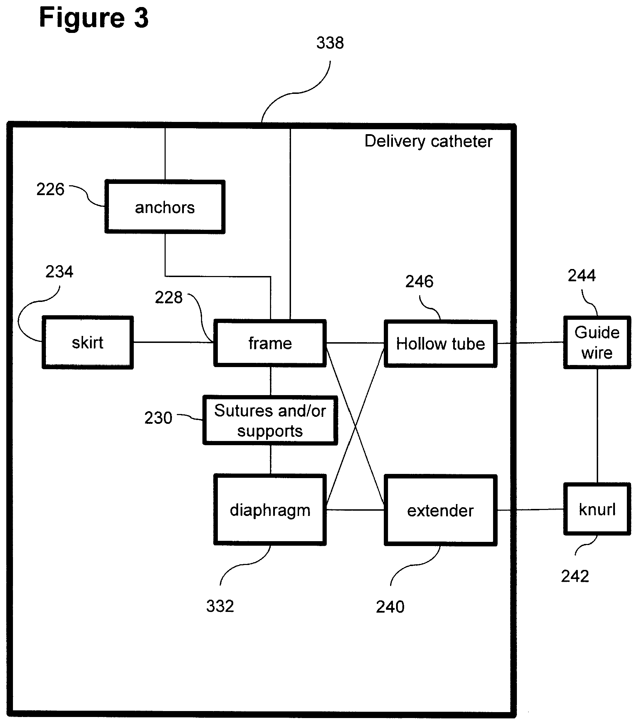

*FIG.* 3 is a block diagram of a prosthetic valve and/or a delivery system in accordance with some embodiments. Optionally a delivery system is used to position and/or deploy a prosthesis in a patient. For example, the delivery system may include a knurl **242** optionally mounted on an extender **240.** The knurl and/or extender optionally prevent premature release of the prosthesis and/or a hollow tube **246.**

In some embodiments, the prosthesis is delivered to a treatment zone using a catheter **338.** Optionally a hollow tube **246** and/or a guide wire **244** and/or an extender **240** are strung from the proximal end of the catheter, through one or more flow channels **266** of the prosthesis (for example between a frame **228** of the prosthesis and a diaphragm **332** of the prosthesis). The prosthesis may for example be collapsed around the tube **246** and/or a guide wire **244** and/or an extender **240** and/or inserted into the catheter. Extender **240** and/or guide wire **244** optionally extend out of the distal and of catheter **338.** Extender optionally holds a knurl **242** distal to catheter **338.** For example, knurl **242** may be a delivery tip of catheter **338.** Optionally, guide wire **244** passes through an opening in knurl **242.** For example, the distal end of guide wire **244** may protrude from a distal opening in knurl **242.** Optionally guide wire **244** may be used to guide catheter **338** to a treatment location. Then guide wire **244** and/or tube **246** and extender **240** and/or knurl **242** may be retrieved (for example using catheter **338)** from the treatment location.

### Functioning of a prosthetic valve

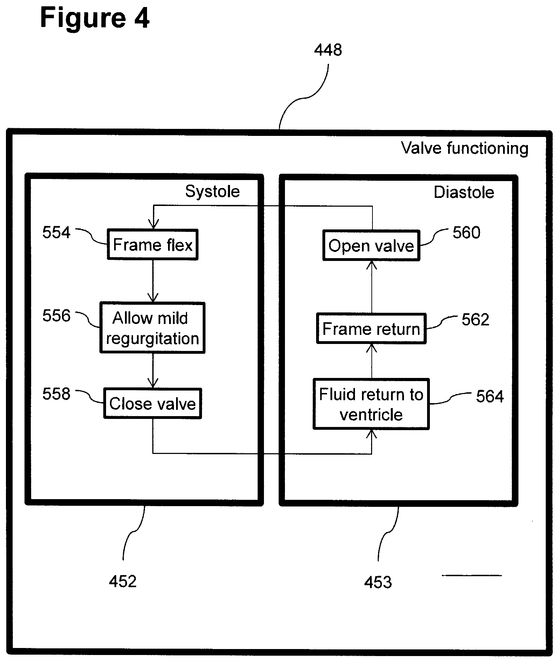

*FIG.* 4 is a flow chart illustrated functioning **448** of a prosthetic valve in accordance with some embodiments. In some embodiments, the valve may permit the annulus to flex while controlling flow through a channel, an orifice and/or annulus. For example the valve may permit one way flow. Optionally, the valve allows mild and/or controlled regurgitation.

In some embodiments, a frame of the valve may take the form of a native annulus. For example as the annulus flexes the frame may flex and/or elastically deform. For example, the ratio of lengths of an axis of the frame and/or annulus before and after deformation may range between 9:10 to 4:5 and/or 4:5 to 2:3 and/or 2:3 to 1:2 and/or 1:2 to 1:4. For example, in the case of a tricuspid valve prosthesis, during diastole **453** the frame may have a nearly circular cross section. Optionally, during systole **452** the frame may flex **554** to an oval shape (for example with a ratio of lengths of a principle and secondary axis ranging between 9:10 to 4:5 and/or 4:5 to 2:3 and/or 2:3 to 1:2 and/or 1:2 to 1:4).

In some embodiments, the valve may close and/or inhibit **558** flow in one direction. For example in the case of a tricuspid prosthetic flow may be blocked and/or inhibited from the right ventricle to the right atrium. Optionally a free edge of the diaphragm may be held pressed to the periphery of the orifice (for example along an inner wall of the frame) by fluid pressure and/or close **558** the valve. Optionally an attachment between the frame and the diaphragm may prevent the diaphragm from being pushed through the orifice.

In some embodiments the valve may allow **556** controlled and/or mild regurgitation. For example, a valve that is connected to a chamber may allow **556** regurgitation of a volume ranging between 1 to 10% of the volume of the chamber and/or between 10 to 15% and/or between 15 to 25% and/or between 25 to 40%. For example in the case of a tricuspid prosthetic, the chamber may be the right ventricle. For example, limited regurgitation flow may be permitted from the right ventricle in the direction of the right atrium. For example in the case of a tricuspid prosthetic, the regurgitated volume may range between 1 to 10 ml and/or between 10 to 15 ml and/or between 15 to 25 ml and/or between 25 to 40 ml.

In some embodiments, regurgitated fluid may be returned **564** to the original chamber. For example, for a tricuspid prosthetic some or all (for example ranging from 10 to 50% and/or from 50 to75 % and/or from 75 to 100%) of the fluid regurgitated from the ventricle during systole **452** may be returned back to the ventricle during diastole **453.** For example, regurgitated fluid may be returned **564** to the ventricle at the beginning of diastole **453.**

In some embodiments the shape of the valve may open **560** for example during diastole allowing flow from a right atrium to a right ventricle during diastole. A frame and/or the annulus of flow may be reformed **562.** For example in a tricuspid prosthetic, the frame and/or tricuspid annulus may reform to an approximately circular cross section during diastole **453.**

### Deployment

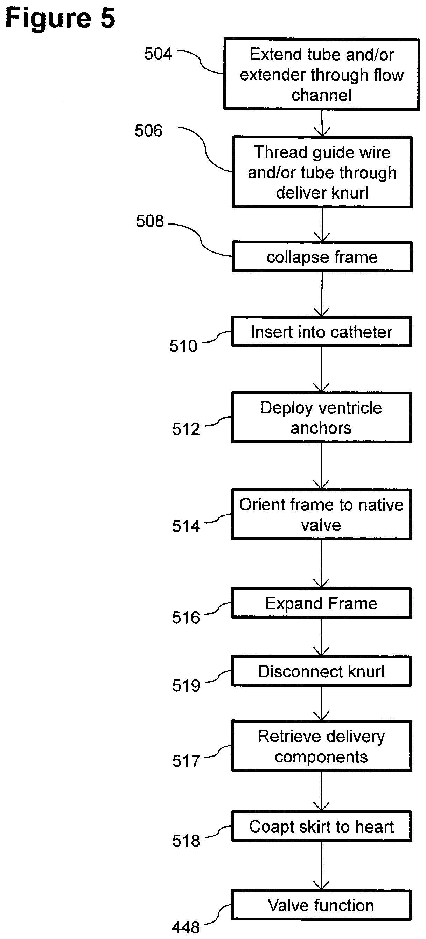

*FIG.* 5 is a flow chart illustrated deployment of a prosthetic valve in accordance with some embodiments. In some embodiments a hollow tube may be extended **504** through a first flow channel of the prosthesis and/or an extender may be extended **504** through another channel. Alternatively and/or additionally, both the tube and/or the extended may be extended **504** through the same channel. For example a knurl may be supported on the distal side of the prosthesis by the extender. Optionally a guide wire and/or the hollow tube and/or the extender may be threaded **506** through the tube and/or an opening in the knurl. For example the tip of the guide wire may extend distally to the knurl.

In some embodiments, the frame may be collapsed **508** around the tube and/or the extender and/or the frame, tube, extender, knurl and/or guide wire may be inserted **510** into a catheter. Optionally the catheter may be used to deliver the prosthesis and/or delivery system to the treatment location (for example the right ventricle of the heart). In the heart, the ventricle stabilizers may optionally be deployed **512.** Optionally the stabilizers and/or the prosthesis may be oriented **514** to the native valve and/or orifice. For example, the ventricle stabilizers may be fit over the native leafs of the valve.

In some embodiments, once the stabilizers are in place, the frame may be expanded **516.** For example, expanding **516** the frame may include attaching the frame to heart tissue. With the frame attached to the heart, the guide wire and/or tube and/or extender may be pulled **519** out of and/or disconnected from the delivery knurl and the guide wire, knurl, extender and/or catheter may be retrieved **517** from the patient. Optionally a skirt and/or ring may be coapted **518** to the heart of the patient. One the valve is in place it may optionally begin to function **448.**

### Views of embodiments of a tricuspid prosthetic

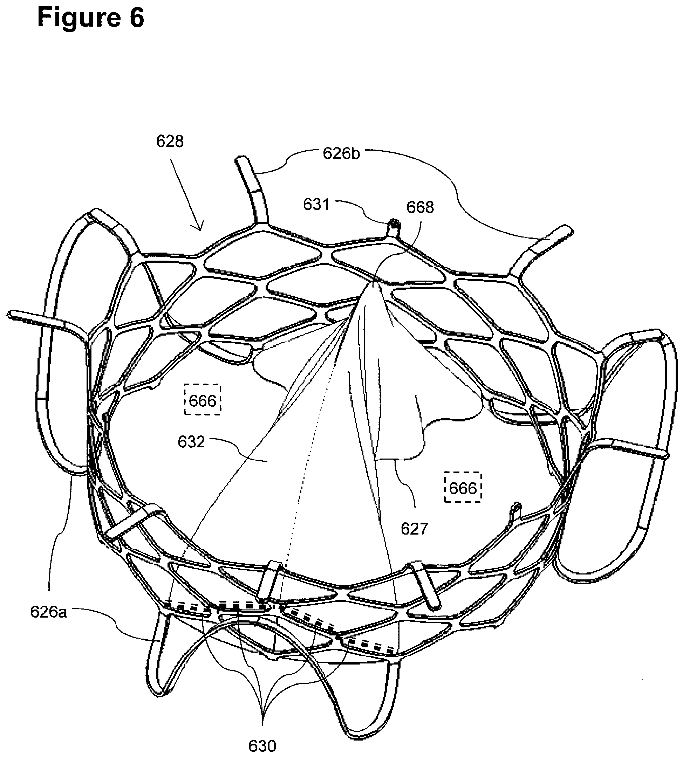

*FIG.* 6 is a perspective illustration of a prosthetic valve with the frame expanded and the valve open in accordance with the current invention. In some embodiments a tricuspid prosthetic includes a central diaphragm **632.** Diaphragm **632** is attached along part of the periphery of a tricuspid annulus to a frame **628.** The attachment is by means of sutures **630.** Optionally diaphragm **632** includes an apex **668.** Optionally apex **668** is located upstream of a frame **628** of the prosthesis.

In some embodiments in the open configuration diaphragm **632** is collapsed and/or folded and/or pushed inward leaving peripheral flow channels **666.** For example flow channels **666** may be between diaphragm **632** and the tricuspid annulus and/or frame **628** and/or an optional skirt. For example, flow channels **666** may be located on opposite sides of diaphragm **632.** Optionally, the sum of the cross sectional areas of the peripheral flow channels **666** may range from 10 to 30% and/or 30 to 50% and/or from 50 to75% and/or from 75 to 100% of the cross section of the tricuspid annulus. For example, in some embodiments, the area flow channels in the open valve may be approximated by slightly (0 - 20%) less than the area of two segments of the circle formed by chords connected the ends of the attached portion of the periphery. For example, for an approximately circular frame of diameter 45 mm having a attachment length of 5 mm, the open area of the flow channels may be between 80 to 95% of the area of the orifice. For example, for an approximately circular frame of diameter 45 mm having a attachment length of 2.5 mm, the open area of the flow channels may be between 85 to 96% of the area of the orifice.

In some embodiments a prosthetic valve may include a coaptation band. For example the edge of the coaptation band may coapt with the back wall of the heart tissue. For example the coaptation band may coapt with the inner wall of the ventricle and/or the inner wall of the atrium. Optionally may include a coaptation band. For example, the width of the coaptation band may range between 0.1 to 2 mm and/or between 2 to 4 mm and/or between 4 to 6 mm and/or between 6 to 10mm and/or between 10 to 15 mm. Optionally the coaptation band may be connected to a skirt may that covers the inner surface of the frame. For example the skirt may cover an area ranging from 0 to 25% and/or from 25 to 50% and/or from 50 to 75% and/or from 75 to 100% of the inner walls of the frame and/or the orifice. Optionally the skirt may be attached to the frame. For example, the skirt may be sutured to the frame. For example the skirt and/or the coaptation band may be made of biocompatible fabric and/or bovine/porcine pericardium.

The diaphragm **632** is attached to a periphery of the frame **628.** The attachment is by means of sutures **630.** The attachment holds diaphragm **632** at least partially expanded and/or open during diastole. Attachment is optionally along struts of frame **628.** Alternatively or additionally diaphragm **632** may be sutured to the skirt. For example, the length of attachment and/or suture lines (suturing the leaflet to the frame and/or the skirt and/or the periphery of the orifice) may range between 0.1 to 1% and/or between 1% to 3% and/or between 3% to 5% and/or between 5% to 7% and/or between 7% to 10% and/or between 10% to 15% and/or between 15% to 30% and/or between 30% to 50% of the perimeter of the entire orifice for example the tricuspid annulus. Optionally, there may be one or more suture lines. For example there may be 2 or 3 suture lines.

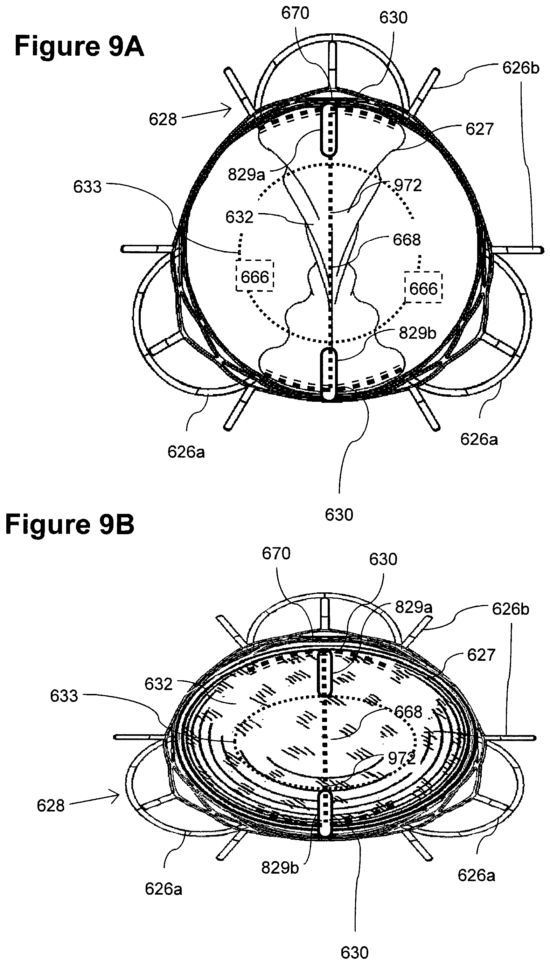

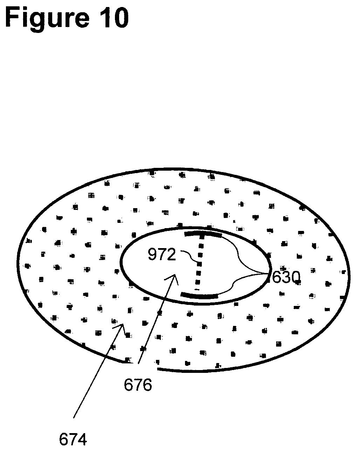

The leaflet is sutured not only along the perimeter of the frame but also in axial direction. Sutures hold a portion of diaphragm **632** partially expanded and/or open during diastole. For example at the beginning of systole, flow into the portion of diaphragm **632** that was expanded and/or open during diastole may cause diaphragm **632** to fill up with blood. In some embodiments, when diaphragm **632** fills, a free edge **627** of diaphragm **632** may be pushed against the periphery of the orifice and/or frame, optionally blocking back flow (for example as illustrated in *FIG.s 6B,* *8B**,* *9B**,* *9D* *and*/*or 9E).* Sutures may optionally be made of a biocompatible material. Optionally the free edge **627** of diaphragm **632** may extend beyond (for example downstream into the ventricle end of) the frame **628.** For example, free edge **627** may extend beyond the frame where there are no sutures. The orifice may in some cases flex to a non-circular shape (for example an oval and/or crescent shape) during systole, the suture lines are optionally positioned substantially perpendicular to the minor axis of the non-circular shape (for example as illustrated in *FIGs. 9B* and/or *10*)*.*

Optionally frame **628** may be made of a self expanding and/or shape memory material, for example nitinol. In some embodiments the axial length of the frame may range for example between 10 to 20mm and or between 20 to 30 mm and/or between 30 to 50mm. Frame **628** may be short in a way it would extend into the right ventricle for only a few millimeters for example between 1 and 5 mm and/or between 5 and 10 mm and/or between 10 and 30 mm. For example the frame may not obstruct the right ventricular outflow tract (RVOT).

In some embodiments frame **628** may include one or more atrial extensions **626b.** Extensions **626b** optionally include anchors and/or stabilizers. For example an atrial extension **626b** may be bent to be approximately parallel (for example between +5 to -5 degrees and/or between +5 to +20 degrees and/or between -5 to -20 degrees) to frame cross section. Atrial extension **626b** optionally extends outward between 0.5 to 2 mm and/or between 2 to 8 mm and/or between 8 to 12 mm and/or between 12 to 20mm radially. Frame **628** optionally includes one or more delivery barbs **631.** For example a delivery barb may be used to connect to a string for controlling positioning of the prosthesis during delivery.

In some embodiments frame **628** may include one or more ventricle extensions **626a.** Extensions **626a** optionally include anchors and/or stabilizers. Ventricular extensions **626a** of the frame are optionally bent backwards towards atrial stabilizers **626b** and/or may reach atrial stabilizers **626b.** For example extension **626a** or **626b** and/or stabilizers and/or anchors may press against the native annulus for migration resistance.

In some embodiments the diameter of frame **628** may range for example between 1 to 10 mm and/or between 10 to 15 mm and/or between 15 to 30 and/or between 30 to 45 mm and/or between 45 to 60mm and/or between 60 to 75mm. For example the diameter may be defined as the mean unstressed diameter and/or maximum unstressed diameter and/or the minimum unstressed diameter. The axial length of frame **628** may range for example between 5 to 15mm and/or between 25 to 25mm and/or between 25 to 60mm. Frame **628** wall thickness may range for example between 0.1 to 0.2 mm and/or between 0.2 to 0.5 mm and/or between 0.5 to 0.75 mm.

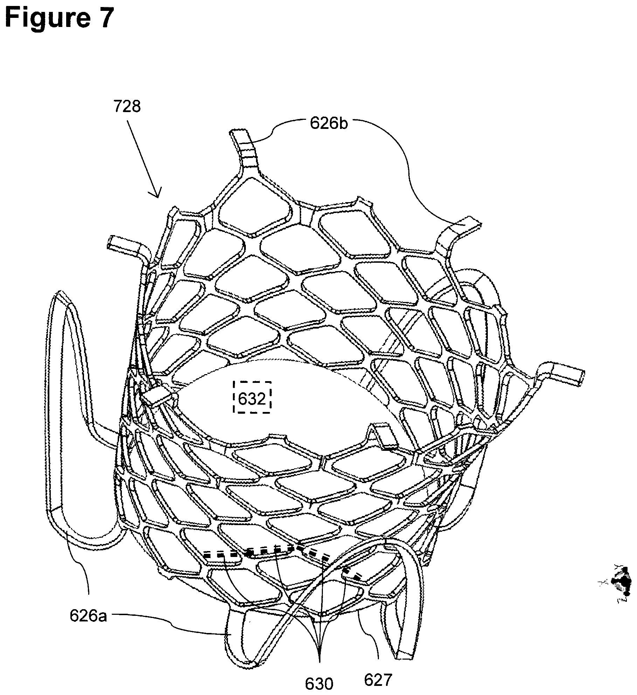

*FIG.* 7 is a perspective illustration of a prosthetic valve with the frame expanded and the valve closed in accordance with some embodiments. Frame **728** may optionally have a longer axial length than frame **628.**

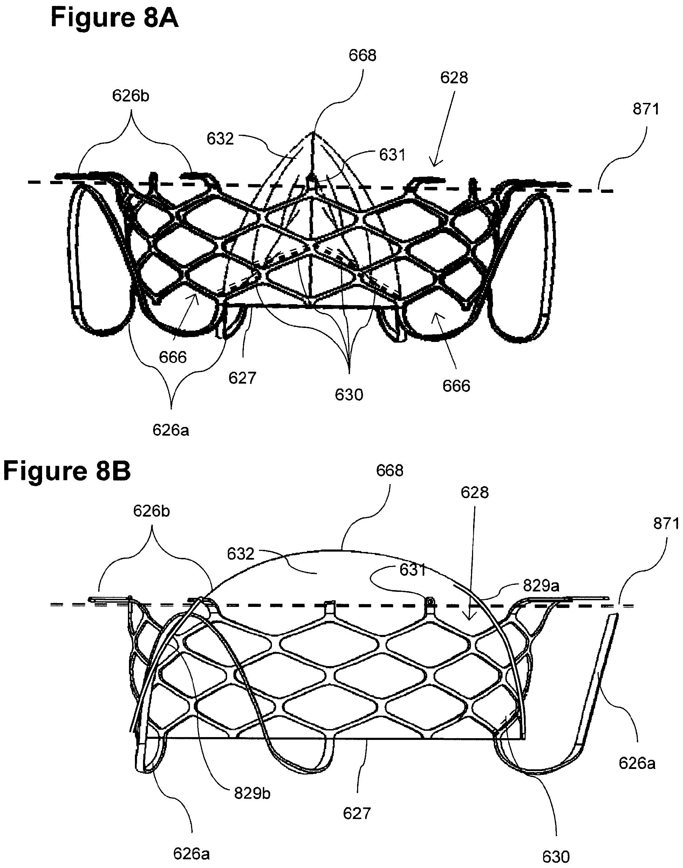

*FIG. 8A* is a side view illustration of a prosthetic valve with the frame expanded and the valve open in accordance with some embodiments. Apex **668** may extend for example a distance ranging from 1 to 10 mm and/or from 10 to 20 mm in the downstream direction into the atrium past the end of frame **628** and/or the tricuspid valve plane **871.** The downstream extension is optionally in a free region of the orifice and/or the projection of the orifice where there is no connection and/or sutures between diaphragm **632** and the frame.

In some embodiments the connection between the diaphragm and the frame is near the edge of the diaphragm. For example, the distance between the edge of the diaphragm and the connection to the frame may be less than half the axial length of the frame and/or less than the axial length of the frame and/or less than 1.5 times the axial length of the frame and/or less than 2 times the axial length of the frame. For example, the distance between the edge of the diaphragm and the connection to the frame may be less than 5mm and/or between 5 to10 mm and/or less than between 10 to 20 mm and/or between 20 to 30 mm and/or between 30 to 40 mm.

*FIG. 8B* is a side view illustration of a prosthetic valve with the frame expanded and the valve open in accordance with some embodiments. Along the inner wall of the frame, diaphragm **632** may be nearly tangential to frame **628.**

In some embodiments, the diaphragm suturing to the frame forms an almost tangential angle, which may in some embodiments reduce the radial forces acting on the frame during systole. The frame optionally has reduced wall thickness may be more compliant to the valve's shape. For example frame **628** may be flexible enough to deform from a circular shape to a crescent and/or oval shape of the tricuspid annulus during diastole and systole. For example during systole, frame may take a elliptical and/or crescent shape and/or during diastole frame may take a more rounded shape. In some embodiments the reduced wall thickness may result in less force exerted on the septal wall of the heart.

The back pressure generated while the valve closes is optionally translated into forces which are exerted on the frame of the valve. The resolved components (axial and radial) may depend on the angle formed between the frame and the diaphragm. For example when the frame and diaphragm are almost tangential the small angle may lead to small radial and/or axial component.

In some embodiments the radial force applied on the native tricuspid annulus may be much less than the force applied to the frame by for example between 10 to 50% and/or between 50 to 75 % and/or between 75 to 90% and/or between 90 to100%. In some embodiments, the outward force helps prevent valvular leakage. The expanding diaphragm optionally adds volume to the ventricle during systole. Added volume may sometimes reduce pressure overload.

In some embodiments, state, apex **668** may for a smooth and/or convex curve.

*FIG. 9A* is a top view illustration of a prosthetic valve with the frame expanded and the valve open in accordance with some embodiments. A flattened portion **670** of frame **628** is optionally near a septal wall of the heart and/or septal ventricle stabilizer **626a** that optionally goes over septal leaflet. This feature may optionally reduce the radial force acting on the septal conduction area of a heart.

In some embodiments, the frame **628** may include one or more support elements, for example element **829a** and/or support element **829b.** Support elements **829a** and **829b** optionally extend from the periphery of the frame and/or orifice into the orifice and/or into the projection of the orifice into the atrium and/or upstream of the orifice and/or into the inflow zone of the orifice in the atrium. Optionally, support elements **829a** and **829b** have a cantilever geometry (for example as can be seen in the side view of support elements **829a** and **829b** illustrated in *FIG. 8B**),* for example only one side of the support element may be attached to the periphery of the frame **628** and/or periphery of the orifice while the other side projects into the flow domain. Optionally support element **829a** extend upstream of the peripheral portion of the frame **628** into the projection of the orifice into the atrium and/or upstream of the orifice and/or into the inflow zone of the orifice in the atrium (for example as is seen in *FIG.* 8B). Optionally support element **829b** is surrounded by and/or is surrounded by and/or within the orifice and/or within the peripheral portion of the frame **628.** Optionally diaphragm **632** is attached (for example by sutures) to support elements **829a** and **829b.**

In some embodiments a region **633** of the flow orifice may be free from frame elements and/or rigid supports and/or rigid elements. For example, region **633** of the exemplary embodiment of FIG. 9A*-D* does not include any stiff structural elements (for example no metal cross piece and/or no self supporting crosspiece except for the diaphragm itself). Optionally there may be wires and/or stitching that stiffens the diaphragm. For example region **633** is a central portion of the orifice. For example, the cross sectional area of region **633** is approximately 40% of the cross sectional area of the orifice. For example, a frame element free region **633** may include the center of the flow region and/or the center of the frame and/or the center of the orifice. For example, a frame element free region **633** may include an area ranging between 10-30% of the cross sectional area of a frame and/or between 30 to 50% and/or between 50 to 80% and/or between 80 to 100%.

In some embodiments, strengthening may be added along all and/or part of minor axis **972.** For example strengthening may include sutures and/or another element.

*FIG. 9B* is a top view illustration of a prosthetic valve with the frame expanded and the valve closed in accordance with some embodiments. For example, in *FIG. 9B**,* frame **628** has flexed to an oval shape. For example the oval shape may conform to native deformation of the tricuspid annulus during systole, for example as illustrated in *FIG. 10**.*

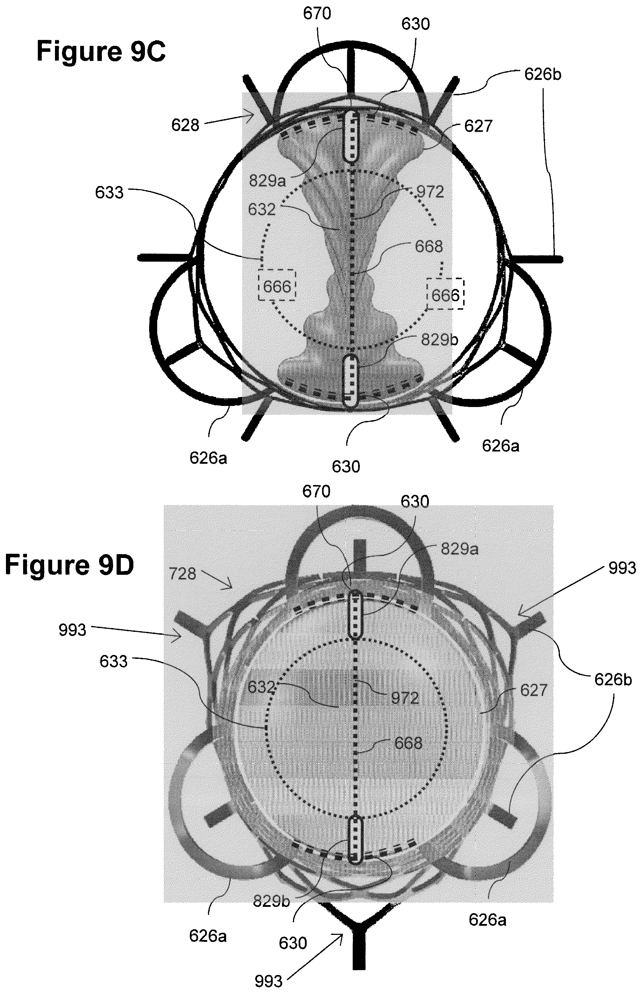

In some embodiments, a prosthetic valve may be designed to function independently of annulus geometries. For example, a valve may be designed to close to backflow to substantially the same degree for an elliptical annulus geometry (for example as illustrated in *FIG. 9B**)* and/or a round annulus geometry (for example as illustrated in *FIG. 9D**)* and/or for a crescent geometry. For example, a valve may close due to pressure forces pushing flexible diaphragm **332** against the inner wall of frame **728.** Optionally pressure may be distributed evenly throughout the blood. In some embodiments, the closing may not be dependent on the geometric form and/or nonuniform bending of a solid element. For example under a fluid pressure differential of 10 to 25 mmHg greater pressure on the ventricle side of the valve, the leakage may be within 0 to 2% and/or **2** to 10% and/or **10** to 30% and/or **30** to 50% and/or **50** to 100% for a round annulus and/or for an annulus with an aspect ratio of 3:4. For example under a fluid pressure differential of 10 to 25 mmHg greater pressure on the ventricle side of the valve, the pressure on the annulus and/or the inner wall of frame **728** be evenly distributed within 0 to 2% and/or **2** to 10% and/or **10** to 30% and/or **30** to 50% and/or **50** to 100% for a round annulus and/or for an annulus with an aspect ratio of 3:4.

*FIG.* 9C is a top view illustration of a prosthetic valve with the frame expanded and the valve open in accordance with some embodiments. Optional supports are shown protruding radially inward along axis **972** from the periphery of frame **628.**

*FIG. 9D* is a top view illustration of a prosthetic valve with the frame expanded and the valve closed in accordance with some embodiments. Frame **728** optionally has circular a cross section on ventricle side and triangle cross section on atrial side. The directions of the triangular vertices of the cross section are marked **993.**

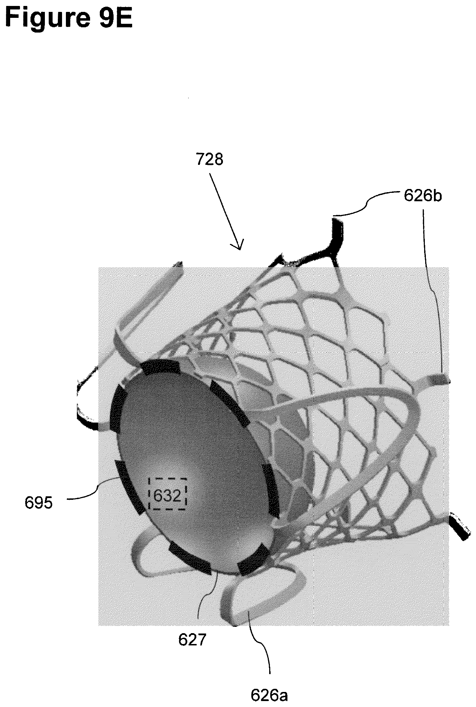

*FIG. 9E* is a perspective view illustration of a prosthetic valve with the frame expanded and the valve closed in accordance with some embodiments. In the expanded state diaphragm **632** optionally has a smooth outer surface. In the expanded state diaphragm **632** optionally has a convex outer surface. For example, the word convex and/or a convex surface may be defined herein in its mathematical sense as a surface having no interior angle greater than 180 degrees and/or as a surface for which any straight line tangent to the surface intersects the surface only once. Examples of diaphragms with smooth, domed and/or convex outer surfaces are illustrated in *FIGS. 7**,* *9B* *and* *9E**.*

In some embodiments (for example as illustrated by the dashed lines in *FIG. 9E**)* the minimal external perimeter **695** and downstream external perimeter of the frame may be on the downstream end of the frame. Perimeter **695** may not include anchors **626a** or extension **626b.** Optionally, a frame may have a thin waist and/or flanged ends. In some embodiments the minimal external perimeter may be around the thin waist. For the sake of the current disclosure a perimeter of a frame may be a closed curve that surrounds the flow channel of the frame.

*FIG. 10* is an illustration of a tricuspid annulus **674** showing positioning of a prosthetic valve in a flow orifice **676** in accordance with some embodiments. For example, as the native valve flexes to an oval or crescent shape during systole. In some embodiments, suture lines **630** may be positioned perpendicular to the minor axis **972** of the oval. For example the suture lines may be at an angle ranging between 85 and 95 degrees of the short axis, and/or between 75 and 105 degrees.

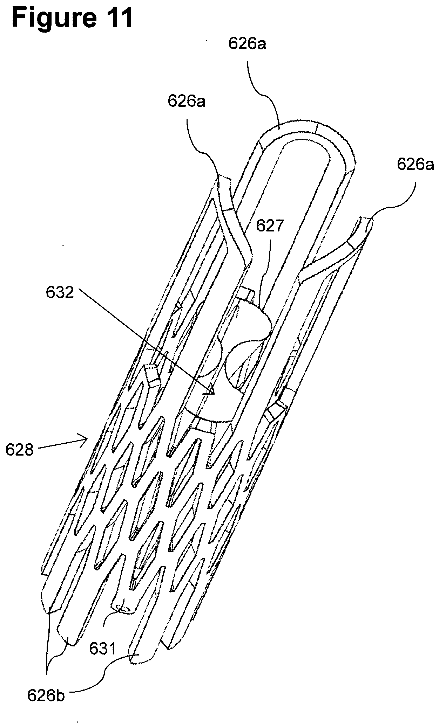

*FIG. 11* is a perspective illustration of a prosthetic valve with the frame **628** collapsed in accordance with some embodiments. In some embodiments, the prosthetic valve in the collapsed configuration may be delivered through a catheter to a treatment region (for example the right ventricle of the heart).

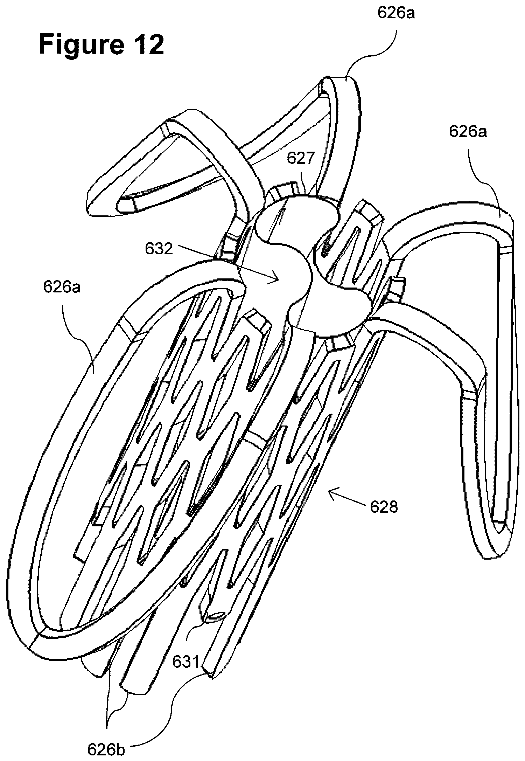

*FIG.* 12 is a perspective illustration of a prosthetic valve with the frame **628** collapsed and ventricle anchors **626a** deployed in accordance with some embodiments. In some embodiment, when a prosthesis arrives at a treatment site in a catheter, the prosthesis may be partially extended from the catheter and anchors **626a** may be deployed. Anchors **626a** are optionally used to orient the prosthesis as it is positioned during deployment. For example, anchors **626a** may be placed over the native leaves of the tricuspid valve. Once the prosthesis is in place the delivery catheter may be gradually retracted for example leaving the prosthesis in place.

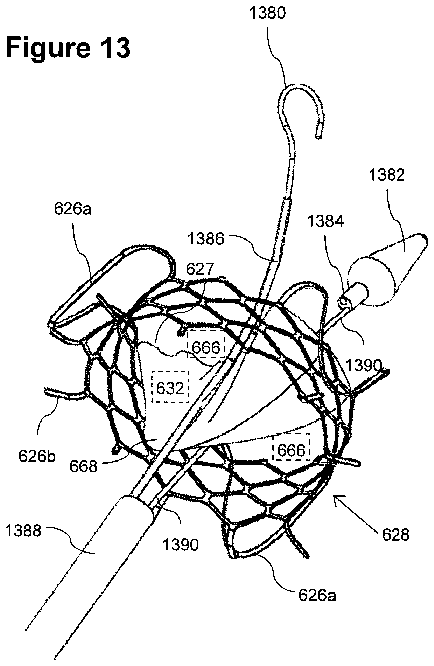

*FIG. 13* is a close up perspective illustration of a prosthetic valve and deployment system with the frame **628** expanded and the valve open in accordance with some embodiments. A guide wire **1380** optionally passes through a tube **1386** which optionally passes through a peripheral channel **666** of the prosthesis. A knurl **1382** includes a proximal opening **1384.** Knurl **1382** is held by an extender **1390.**

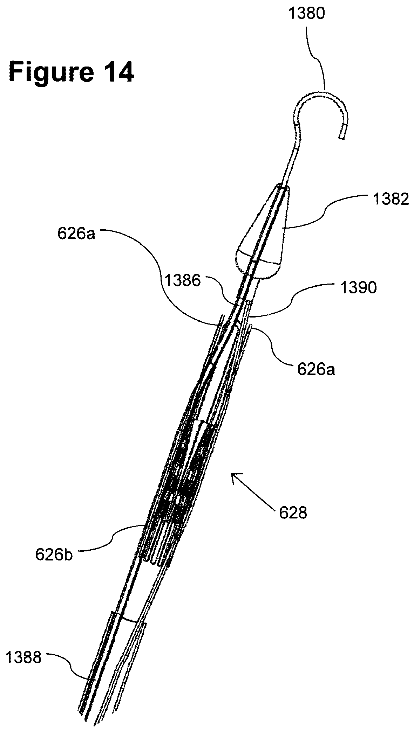

*FIG. 14* is a close up perspective illustration of a prosthetic valve and deployment system with the frame **628** collapsed in accordance with some embodiments. Guide wire **1380** has optionally been passed through knurl **1382** locking tube **1386** and/or the prosthesis to a catheter **1388.** As long as the guide wire is threaded through the tip, the bioprosthesis is optionally held to catheter **1388.** Knurl **1382** is optionally smoothed and/or rounded. The radius of knurl **1382** may range between 0-2 mm and/or from 2 to 5 mm and/or from 5 to 7 mm and/or 7 to 15 mm. The length of knurl **1382** may range between 0 to 4 mm and/or between 5 to 8 mm and/or between 8 to 12mm and between 12 to 15mm and/or between 15 to 25 mm.

In some embodiments, while the prosthesis is in its collapsed state guide wire **1380** and delivery knurl **1382,** may be used to steer the catheter **1388** for example to arrive through an artery to a treatment zone.

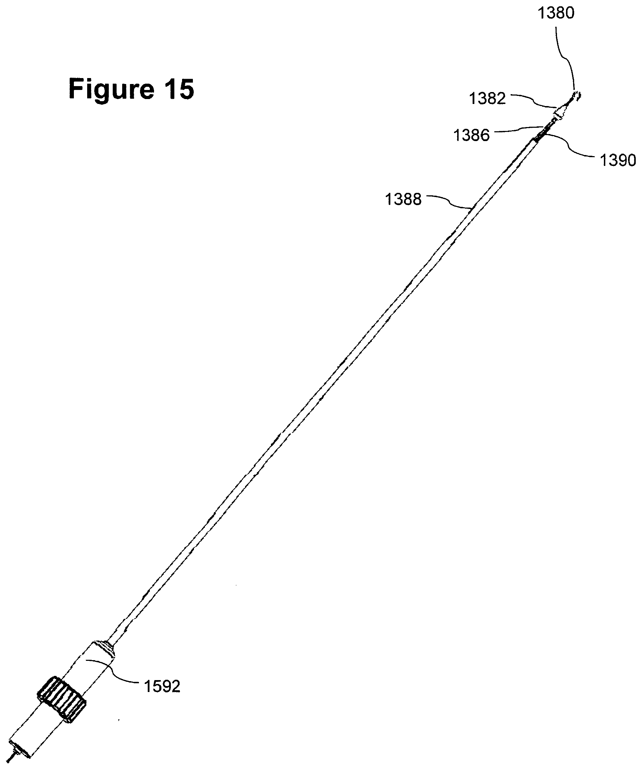

*FIG. 15* is a perspective illustration of a prosthetic valve and deployment system with the frame **628** collapsed in accordance with some embodiments. A catheter controller **1592** is shown. Catheter **1388** diameter may optionally range between 4 to 9 mm (for example 14-28 Fr in the French scale). Delivery system length may optionally range for example between 1 to 25 and/or between 25 to 100 and/or between 100 to 200cm.

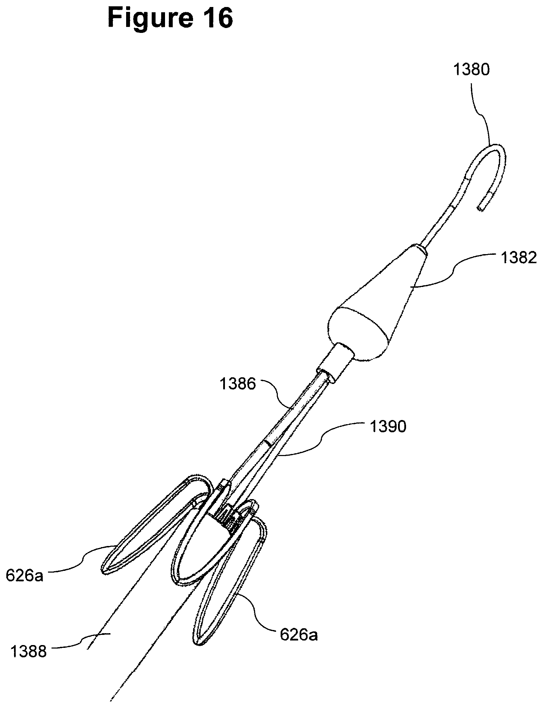

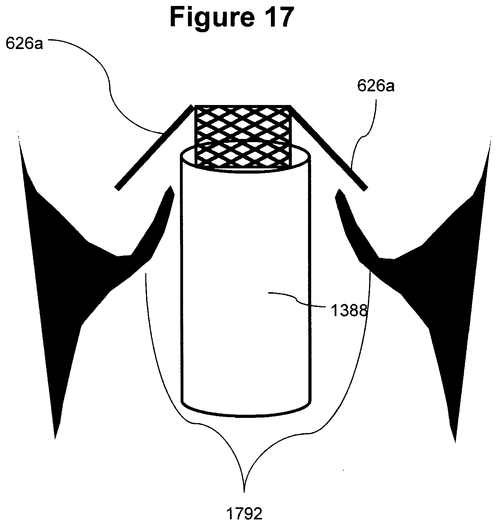

*FIG. 16* is a close up perspective illustration of a prosthetic valve and deployment system with the frame **628** collapsed and ventricle stabilizers **626a** deployed in accordance with some embodiments. For example guide wire **1380** may be inserted through the circulatory system to a right atrium of a patient. Optionally, in the collapsed state (for example as illustrated in *FIG. 15*) the distal portion of the catheter system may be inserted through the right atrium into the tricuspid annulus into the right ventricle. One the system is in the right ventricle, ventricle stabilizers **626a** may be deployed (for example as illustrated in *FIG. 16*)*.* The whole system may optionally be pulled backward (proximally) until ventricle stabilizers **626a** are in position. For example, ventricle stabilizers **626a** may be positioned over the native leaflets of the valve (for example leaflets **1792** as illustrated in *FIG. 17**).* Once stabilizers **626a** are positioned, the delivery catheter **1388** may optionally be pulled away and/or frame **628** may be expanded into the tricuspid annulus. With the valve in place guide wire **1380** may be detached from the knurl **1382** and/or delivery catheter **1388** is optionally pulled back out of the patient for example with the guide wire **1380,** tube **1386,** extender **1390** and/or delivery knurl **1382** leaving the prosthesis in place.

*FIG. 17* is a close up perspective illustration of deployment of a prosthetic valve in accordance with some embodiments. Ventricle stabilizers **626a** are shown optionally fit over native leaflets **1792.**

It is expected that during the life of a patent maturing from this application many relevant technologies (for example delivery methods and/or prosthetic materials) will be developed and the scope of the terms are intended to include all such new technologies *a priori.* As used herein the term "about" refers to ± 5%

The terms "comprises", "comprising", "includes", "including", "having" and their conjugates mean "including but not limited to".

The term "consisting of" means "including and limited to".

The term "consisting essentially of" means that the composition, method or structure may include additional ingredients, steps and/or parts, but only if the additional ingredients, steps and/or parts do not materially alter the basic and novel characteristics of the claimed composition, method or structure.

As used herein, the singular form "a", "an" and "the" include plural references unless the context clearly dictates otherwise. For example, the term "a compound" or "at least one compound" may include a plurality of compounds, including mixtures thereof.

Throughout this application, various embodiments may be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

Whenever a numerical range is indicated herein, it is meant to include any cited numeral (fractional or integral) within the indicated range. The phrases "ranging/ranges between" a first indicate number and a second indicate number and "ranging/ranges from" a first indicate number "to" a second indicate number are used herein interchangeably and are meant to include the first and second indicated numbers and all the fractional and integral numerals therebetween.

As used herein the term "method" refers to manners, means, techniques and procedures for accomplishing a given task including, but not limited to, those manners, means, techniques and procedures either known to, or readily developed from known manners, means, techniques and procedures by practitioners of the chemical, pharmacological, biological, biochemical and medical arts.

As used herein, the term "treating" includes abrogating, substantially inhibiting, slowing or reversing the progression of a condition, substantially ameliorating clinical or aesthetical symptoms of a condition or substantially preventing the appearance of clinical or aesthetical symptoms of a condition.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination or as suitable in any other described embodiment of the invention. Certain features described in the context of various embodiments are not to be considered essential features of those embodiments, unless the embodiment is inoperative without those elements.

Although the invention has been described in conjunction with specific embodiments thereof, it is evident that many alternatives, modifications and variations will be apparent to those skilled in the art. Accordingly, it is intended to embrace all such alternatives, modifications and variations that fall within the scope of the appended claims.

Citation or identification of any reference in this application shall not be construed as an admission that such reference is available as prior art to the present invention. To the extent that section headings are used, they should not be construed as necessarily limiting.

## Claims

1. A prosthetic heart valve system comprising:
a. a prosthetic heart valve configured to be anchored between an atrial side of a valve of a heart and a ventricular side of said valve of said heart, comprising:
i. a frame (628, 728) sized and shaped to fit in an orifice of a heart valve, said frame (628, 728) having an atrial end configured to be placed at said atrial side and a ventricular end configured to be placed at said ventricular side; said frame (628, 728) defining a flow channel (666); said frame (628, 728) having a plurality of atrial extensions (626b) being bent to be approximately parallel to a frame cross section; said plurality of atrial extensions (626b) extending outward between 0.5 mm to 20 mm radially;
ii. a flexible diaphragm (632); said flexible diaphragm (632) is connected to the frame (628, 728) at two locations using a plurality of sutures lines (230); said flexible diaphragm (632) comprises an open configuration and a closed configuration; wherein in said open configuration two sides of said flexible diaphragm (632) collapse toward the center of said prosthetic heart valve; and
b. a delivery system for said prosthetic heart valve, comprising:
i. an extender (240, 1390) including a first portion with a proximal end located proximal to the prosthetic heart valve, the first portion extending through said flow channel (666) of the prosthetic heart valve and a second portion located distal to the prosthetic heart valve;
ii. a delivery tip (242) mounted on the second portion of the extender (240, 1390);
iii. a catheter (338, 1388) configured to fit said prosthetic heart valve in a collapsed configuration;
**characterized in**
said plurality of suture lines (230) extending in both a circumferential and axial direction thereby holding a portion of said diaphragm partially expanded.

2. The prosthetic heart valve system according to claim 1, wherein said frame (628, 728) comprises a length ranging between 15 mm to 45 mm.

3. The prosthetic heart valve system according to claim 1 or claim 2, wherein said frame (628, 728) comprises a plurality of anchors (626a) on said ventricular end of said frame.

4. The prosthetic heart valve system according to any one of claims 1-3, wherein said plurality of atrial extensions (626b) extend outward between 8 mm to 12 mm radially.

5. The prosthetic heart valve system according to any one of claims 1-4, wherein in said open configuration said flexible diaphragm (632) is collapsed.

6. The prosthetic heart valve system according to any one of claims 1-5, wherein in said closed configuration a free edge of said flexible diaphragm (632) expands against a periphery of said frame (628, 728).

7. The prosthetic heart valve system according to any one of claims 1-6, wherein in said closed configuration a free edge of said flexible diaphragm (632) expands against a periphery of a skirt (234) connected to said frame (628, 728).

8. The prosthetic heart valve system according to any one of claims 1-7, wherein said flexible diaphragm (632) having a length of an edge, said length of an edge being larger than the perimeter of said flow channel (666) of said frame (628, 728); and
wherein said length of an edge being larger in a range between 50% to 100% than the perimeter of said flow channel (666) of said frame (628, 728).

9. The prosthetic heart valve system according to any one of claims 1-8, wherein the frame (628, 728) has a downstream border and wherein the edge of the flexible diaphragm (632) is at least as large as a perimeter of the frame (628, 728) at the downstream border.

10. The prosthetic heart valve system according to any one of claims 1-9, wherein the frame (628, 728) surrounds a connected space and the flexible diaphragm (632) divides the connected space into at least two disconnected channels.

11. The prosthetic heart valve system according to any one of claims 1-10, wherein the flexible diaphragm (632) has a collapsed position allowing flow from an upstream direction to a downstream direction between the flexible diaphragm (632) and the perimeter of the orifice and an expanded position inhibiting the flow; and
wherein said prosthetic heart valve is configured to switch from said collapsed position to said expanded position in response to a back flow from a downstream chamber, said back flow occurring between a beginning of a systolic phase until said flexible diaphragm (632) reaches said expanded position and wherein said prosthetic heart valve is further configured to permit a back flow volume of between 1 to 40 % of a volume of said downstream chamber to flow from said downstream chamber into at least one of a volume of said flexible diaphragm (632) and an upstream chamber.

12. The prosthetic heart valve system according to any one of claims 1-11, wherein there is no stiff element connected on two sides to the frame (628, 728).

13. The prosthetic heart valve system according to any one of claims 1-12, wherein said flow channel (666) does not include a stiff self-supporting crosspiece; and wherein said flow channel (666) in a portion thereof including the flexible diaphragm (632) does not include a self-supporting crosspiece other than said flexible diaphragm (632).

14. The prosthetic heart valve system according to any one of claims 1-13, wherein said extender (240, 1390) prevents premature release of said prosthetic heart valve.

15. The prosthetic heart valve system according to any one of claims 1-14, wherein said catheter (338, 1388) prevents premature release of said prosthetic heart valve while said prosthetic heart valve is fitted inside said catheter (338, 1388).

16. The prosthetic heart valve system according to any one of claims 1-15, wherein said delivery tip is configured to let a guidewire pass through an opening in said delivery tip.

## Patentansprüche

1. Herzklappenprothesensystem, umfassend:
a. eine Herzklappenprothese, die dazu konfiguriert ist, zwischen einer atrialen Seite einer Klappe eines Herzens und einer ventrikulären Seite der Klappe des Herzens verankert zu sein, umfassend:
i. einen Rahmen (628, 728), der so dimensioniert und geformt ist, dass er in einen Eingang einer Herzklappe passt, wobei der Rahmen (628, 728) ein atriales Ende, das dazu konfiguriert ist, auf der atrialen Seite platziert zu sein, und ein ventrikuläres Ende, das dazu konfiguriert ist, auf der ventrikulären Seite platziert zu sein, aufweist; wobei der Rahmen (628, 728) einen Strömungskanal (666) definiert; wobei der Rahmen (628, 728) eine Vielzahl von atrialen Erstreckungen (626b) aufweist, die so gebogen sind, dass sie etwa parallel zu einem Rahmenquerschnitt sind; wobei die Vielzahl von atrialen Erstreckungen (626b) sich zwischen 0,5 mm und 20 mm radial nach außen erstrecken;
ii. eine flexible Membran (632); wobei die flexible Membran (632) unter Verwendung einer Vielzahl von Nahtlinien (230) an zwei Stellen mit dem Rahmen (628, 728) verbunden ist; wobei die flexible Membran (632) eine offene Konfiguration und eine geschlossene Konfiguration umfasst; wobei in der offenen Konfiguration zwei Seiten der flexiblen Membran (632) zu der Mitte der Herzklappenprothese hin zusammenklappen; und
b. ein Zuführsystem für die Herzklappenprothese, umfassend:
i. eine Erstreckungsvorrichtung (240, 1390), die einen ersten Abschnitt mit einem proximalen Ende, das proximal zu der Herzklappenprothese angeordnet ist, wobei sich der erste Abschnitt durch den Strömungskanal (666) der Herzklappenprothese erstreckt, und einen zweiten Abschnitt, der distal zu der Herzklappenprothese angeordnet ist, beinhaltet;
ii. eine Zuführspitze (242), die an dem zweiten Abschnitt der Erstreckungsvorrichtung (240, 1390) angebracht ist;
iii. einen Katheter (338, 1388), der dazu konfiguriert ist, die Herzklappenprothese in einem zusammengeklappten Zustand einzupassen; **gekennzeichnet dadurch, dass**
sich die Vielzahl von Nahtlinien (230) in sowohl eine Umfangsrichtung als auch eine axiale Richtung erstrecken, wodurch ein Abschnitt der Membran teilweise ausgedehnt gehalten wird.

2. Herzklappenprothesensystem nach Anspruch 1, wobei der Rahmen (628, 728) eine Länge umfasst, die in einem Bereich zwischen 15 mm und 45 mm liegt.

3. Herzklappenprothesensystem nach Anspruch 1 oder Anspruch 2, wobei der Rahmen (628, 728) eine Vielzahl von Ankern (626a) an dem ventrikulären Ende des Rahmens umfasst.

4. Herzklappenprothesensystem nach einem der Ansprüche 1 bis 3, wobei die Vielzahl von atrialen Erstreckungen (626b) sich zwischen 8 mm und 12 mm radial nach außen erstrecken.

5. Herzklappenprothesensystem nach einem der Ansprüche 1 bis 4, wobei die flexible Membran (632) in der offenen Konfiguration zusammengeklappt ist.

6. Herzklappenprothesensystem nach einem der Ansprüche 1 bis 5, wobei sich eine freie Kante der flexiblen Membran (632) in der geschlossenen Konfiguration gegen eine Umrandung des Rahmens (628, 728) ausdehnt.

7. Herzklappenprothesensystem nach einem der Ansprüche 1 bis 6, wobei sich eine freie Kante der flexiblen Membran (632) in der geschlossenen Konfiguration gegen eine Umrandung einer mit dem Rahmen (628, 728) verbundenen Blende (234) ausdehnt.

8. Herzklappenprothesensystem nach einem der Ansprüche 1 bis 7, wobei die flexible Membran (632) eine Länge einer Kante aufweist, wobei die Länge einer Kante größer ist als die Umrandung des Strömungskanals (666) des Rahmens (628, 728); und
wobei die Länge einer Kante in einem Bereich zwischen 50 % und 100 % größer ist als die Umrandung des Strömungskanals (666) des Rahmens (628, 728).

9. Herzklappenprothesensystem nach einem der Ansprüche 1 bis 8, wobei der Rahmen (628, 728) eine stromabwärtige Begrenzung aufweist und wobei die Kante der flexiblen Membran (632) mindestens so groß wie eine Umrandung des Rahmens (628, 728) an der stromabwärtigen Begrenzung ist.

10. Herzklappenprothesensystem nach einem der Ansprüche 1 bis 9, wobei der Rahmen (628, 728) einen verbundenen Raum umgibt und die flexible Membran (632) den verbundenen Raum in mindestens zwei unverbundene Kanäle aufteilt.

11. Herzklappenprothesensystem nach einem der Ansprüche 1 bis 10, wobei die flexible Membran (632) eine zusammengeklappte Position, die eine Strömung von einer stromaufwärtigen Richtung zu einer stromabwärtigen Richtung zwischen der flexiblen Membran (632) und der Umrandung des Eingangs ermöglicht, und eine ausgedehnte Position, die die Strömung blockiert, aufweist; und
wobei die Herzklappenprothese dazu konfiguriert ist, als Antwort auf eine Rückströmung von einer stromabwärtigen Kammer von der zusammengeklappten Position in die ausgedehnte Position zu wechseln, wobei die Rückströmung zwischen einem Beginn einer systolischen Phase auftritt, bis die flexible Membran (632) die ausgedehnte Position erreicht, und wobei die Herzklappenprothese ferner dazu konfiguriert ist, zuzulassen, dass ein Rückströmungsvolumen zwischen 1 und 40 % eines Volumens der stromabwärtigen Kammer von der stromabwärtigen Kammer in mindestens eines von einem Volumen der flexiblen Membran (632) und einer stromaufwärtigen Kammer strömt.

12. Herzklappenprothesensystem nach einem der Ansprüche 1 bis 11, wobei es kein steifes Element gibt, das auf zwei Seiten mit dem Rahmen (628, 728) verbunden ist.

13. Herzklappenprothesensystem nach einem der Ansprüche 1 bis 12, wobei der Strömungskanal (666) kein steifes selbsttragendes Querstück beinhaltet; und wobei der Strömungskanal (666) in einem Abschnitt davon, der die flexible Membran (632) beinhaltet, kein selbsttragendes Querstück außer der flexiblen Membran (632) beinhaltet.

14. Herzklappenprothesensystem nach einem der Ansprüche 1 bis 13, wobei die Erstreckungsvorrichtung (240, 1390) eine vorzeitige Freisetzung der Herzklappenprothese verhindert.

15. Herzklappenprothesensystem nach einem der Ansprüche 1 bis 14, wobei der Katheter (338, 1388) eine vorzeitige Freisetzung der Herzklappenprothese verhindert, während die Herzklappenprothese im Inneren des Katheters (338, 1388) eingepasst wird.

16. Herzklappenprothesensystem nach einem der Ansprüche 1 bis 15, wobei die Zuführspitze dazu konfiguriert ist, einen Führungsdraht durch eine Öffnung in der Zuführspitze passieren zu lassen.

## Revendications

1. Système de valvule cardiaque prothétique comprenant :
a. une valvule cardiaque prothétique configurée pour être ancrée entre un côté atrial d'une valvule d'un cœur et un côté ventriculaire de ladite valvule dudit cœur, comprenant :
i. une armature (628, 728) dimensionnée et façonnée pour s'adapter à un orifice d'une valvule cardiaque, ladite armature (628, 728) ayant une extrémité atriale configurée pour être placée au niveau dudit côté atrial et une extrémité ventriculaire configurée pour être placée au niveau dudit côté ventriculaire ; ladite armature (628, 728) définissant un canal d'écoulement (666) ; ladite armature (628, 728) ayant une pluralité d'extensions atriales (626b) étant recourbées pour être approximativement parallèles à une section transversale d'armature ; ladite pluralité d'extensions atriales (626b) s'étendant vers l'extérieur entre 0,5 mm et 20 mm radialement ;
ii. une membrane flexible (632) ; ladite membrane flexible (632) est accouplée à l'armature (628, 728) au niveau de deux emplacements en utilisant une pluralité de lignes de sutures (230) ; ladite membrane flexible (632) comprend une configuration ouverte et une configuration fermée ; dans lequel dans ladite configuration ouverte deux côtés de ladite membrane flexible (632) se replient vers le centre de ladite valvule cardiaque prothétique ; et
b. un système de distribution pour ladite valvule cardiaque prothétique, comprenant :
i. un élément d'extension (240, 1390) incluant une première partie avec une extrémité proximale située de manière proximale à la valvule cardiaque prothétique, la première partie s'étendant à travers ledit canal d'écoulement (666) de la valvule cardiaque prothétique et une deuxième partie située de manière distale à la valvule cardiaque prothétique ;
ii. une pointe de distribution (242) montée sur la deuxième partie de l'élément d'extension (240, 1390) ;
iii. un cathéter (338, 1388) configuré pour s'adapter à ladite valvule cardiaque prothétique dans une configuration repliée ;
**caractérisé en ce que**
ladite pluralité de lignes de sutures (230) s'étendant à la fois dans une direction circonférentielle et axiale, maintenant ainsi une partie de ladite membrane partiellement déployée.

2. Système de valvule cardiaque prothétique selon la revendication 1, dans lequel ladite armature (628, 728) comprend une longueur allant de 15 mm à 45 mm.

3. Système de valvule cardiaque prothétique selon la revendication 1 ou la revendication 2, dans lequel ladite armature (628, 728) comprend une pluralité d'ancrages (626a) sur ladite extrémité ventriculaire de ladite armature.

4. Système de valvule cardiaque prothétique selon l'une quelconque des revendications 1 à 3, dans lequel ladite pluralité d'extensions atriales (626b) s'étendent vers l'extérieur entre 8 mm et 12 mm radialement.

5. Système de valvule cardiaque prothétique selon l'une quelconque des revendications 1 à 4, dans lequel dans ladite configuration ouverte, ladite membrane flexible (632) est repliée.

6. Système de valvule cardiaque prothétique selon l'une quelconque des revendications 1 à 5, dans lequel dans ladite configuration fermée, un bord libre de ladite membrane flexible (632) se déploie contre une périphérie de ladite armature (628, 728).

7. Système de valvule cardiaque prothétique selon l'une quelconque des revendications 1 à 6, dans lequel dans ladite configuration fermée, un bord libre de ladite membrane flexible (632) se déploie contre une périphérie d'une jupe (234) accouplée à ladite armature (628, 728).

8. Système de valvule cardiaque prothétique selon l'une quelconque des revendications 1 à 7, dans lequel ladite membrane flexible (632) a une longueur d'un bord, ladite longueur d'un bord étant supérieure au périmètre dudit canal d'écoulement (666) de ladite armature (628, 728) ; et
dans lequel ladite longueur d'un bord est supérieure dans une plage comprise entre 50 % et 100 % au périmètre dudit canal d'écoulement (666) de ladite armature (628, 728).

9. Système de valvule cardiaque prothétique selon l'une quelconque des revendications 1 à 8, dans lequel l'armature (628, 728) a une bordure en aval et dans lequel le bord de la membrane flexible (632) est au moins aussi grand qu'un périmètre de l'armature (628, 728) au niveau de la bordure en aval.

10. Système de valvule cardiaque prothétique selon l'une quelconque des revendications 1 à 9, dans lequel l'armature (628, 728) entoure un espace accouplé et la membrane flexible (632) divise l'espace accouplé en au moins deux canaux désaccouplés.

11. Système de valvule cardiaque prothétique selon l'une quelconque des revendications 1 à 10, dans lequel la membrane flexible (632) a une position repliée permettant un écoulement d'une direction en amont vers une direction en aval entre la membrane flexible (632) et le périmètre de l'orifice et une position déployée inhibant l'écoulement ; et
dans lequel ladite valvule cardiaque prothétique est configurée pour basculer de ladite position repliée à ladite position déployée en réponse à un refoulement à partir d'une chambre en aval, ledit refoulement se produisant entre un début d'une phase systolique jusqu'à ce que ladite membrane flexible (632) atteigne ladite position déployée et dans lequel ladite valvule cardiaque prothétique est en outre configurée pour permettre à un volume de refoulement compris entre 1 à 40 % d'un volume de ladite chambre en aval de s'écouler à partir de ladite chambre en aval dans au moins un d'un volume de ladite membrane flexible (632) et d'une chambre en amont.

12. Système de valvule cardiaque prothétique selon l'une quelconque des revendications 1 à 11, dans lequel il n'existe pas d'élément rigide accouplé sur les deux côtés à l'armature (628, 728).

13. Système de valvule cardiaque prothétique selon l'une quelconque des revendications 1 à 12, dans lequel ledit canal d'écoulement (666) n'inclut pas de traverse autoportante rigide ; et dans lequel ledit canal d'écoulement (666) dans une partie de celui-ci incluant la membrane flexible (632) n'inclut pas de traverse autoportante autre que ladite membrane flexible (632).

14. Système de valvule cardiaque prothétique selon l'une quelconque des revendications 1 à 13, dans lequel ledit élément d'extension (240, 1390) empêche une libération prématurée de ladite valvule cardiaque prothétique.

15. Système de valvule cardiaque prothétique selon l'une quelconque des revendications 1 à 14, dans lequel ledit cathéter (338, 1388) empêche la libération prématurée de ladite valvule cardiaque prothétique alors que ladite valvule cardiaque prothétique est insérée à l'intérieur dudit cathéter (338, 1388).

16. Système de valvule cardiaque prothétique selon l'une quelconque des revendications 1 à 15, dans lequel ladite pointe de distribution est configurée pour laisser passer un fil-guide à travers une ouverture dans ladite pointe de distribution.
